# EUROPÄISCHE PATENTSCHRIFT

(11) **EP 1 266 674 B1**
(45) Veröffentlichungstag und Bekanntmachung des Hinweises auf die Patenterteilung: **20.05.2009**
(21) Anmeldenummer: 02008538.7
(22) Anmeldetag: 16.04.2002
(51) Int. Cl.: A62B 18/08, A61M 16/06

(54) **Vorrichtung zur Abstützung**
Support device
Dispositif d'appui

(30) Priorität: 15.06.2001 DE 10128994
(43) Veröffentlichungstag der Anmeldung: 18.12.2002
(73) Patentinhaber: Weinmann Geräte für Medizin GmbH & Co. KG, 22525 Hamburg (DE)
(72) Erfinder: Eifler, Martin, 25348 Glückstadt (DE)
(74) Vertreter: Klickow, Hans-Henning

(56) Entgegenhaltungen:
- EP-A- 0 634 186
- EP-A- 1 099 452
- WO-A-00/20072
- WO-A-87/01950
- WO-A-99/65554
- US-A- 4 960 121
- US-A- 6 119 693

## Beschreibung

Die Erfindung betrifft eine Vorrichtung zur Abstützung einer Beatmungseinrichtung relativ zu einem Kopfbereich eines Benutzers, die mindestens ein von einem Träger gehaltertes Polsterelement aufweist sowie bei der sich das Polsterelement mit mindestens einem Stützschenkel mindestens bereichsweise seitlich neben einem Außenschenkel des Trägers erstreckt und mindestens bereichsweise mit einem Abstand zum Außenschenkel ein Innenschenkel des Trägers verläuft.

Derartige Polsterelemente werden häufig in einer Ausbildung als elastomeres Polster im Bereich von Stirnstützen, vollgesichtsmasken oder von Nasalmasken verwendet. Die bislang bekannten Polsterelemente können noch nicht alle Anforderungen erfüllen, die sowohl an eine einfache Montierbarkeit als auch an eine hohe Fixiersicherheit bei einer Vielzahl von einwirkenden Benutzungsparametern gestellt werden. Insbesondere tritt bei den bekannten Polsterelementen häufig das Problem auf, daß bei einem Einwirken von Querkräften ein partielles oder vollständiges Lösen des Polsterelementes vom Träger erfolgt, so daß der Benutzer anschließend das Polsterelement wieder manuell in der Ausgangsposition anordnen muß, was insbesondere bei einem ungeübten Benutzer einen erheblichen Zeitaufwand verursacht. Darüber hinaus läßt bei einem mehrfachen Trennen des Polsterelementes vom Träger die Elastizität des Polsterelementes nach und es können nicht reversible Materialaufweitungen entstehen, die zu einer verschlechterten Passung zwischen dem Polsterelement und dem Träger führen.

Aus der US-A-4,960,121 ist bereits eine Vorrichtung zur Abstützung einer Beatmungseinrichtung bekannt, die ein Polsterelement, Stützschenkel, Träger sowie Innenschenkel und Außenschenkel aufweist. Eine ähnliche Vorrichtung ist in der EP-A-1 099 452 beschrieben.

Gleichfalls ist aus der WO 00/20072 A eine vorrichtung mit den oben erwähnten Merkmalen bekannt. Nochmals eine ähnliche Vorrichtung, jedoch ohne Verwendung eines Außenschenkels, wird in der EP-A-0 634 186 erläutert.

Aufgabe der vorliegenden Erfindung ist es daher, eine Vorrichtung der einleitend genannten Art derart zu konstruieren, daß eine verbesserte Fixierung des Polsterelementes im Bereich des Trägers erreicht wird.

Diese Aufgabe wird erfindungsgemäß dadurch gelöst, daß sich zwischen dem Stützschenkel und dem Außenschenkel mindestens bereichsweise ein Abstand erstreckt, daß das Polsterelement eine Nut zur Aufnahme eines Haltesteges des Trägers aufweist, und daß das Polsterelement und der Träger über die Nut und den Haltesteg miteinander verbunden sind.

Durch die Anordnung des Stützschenkels neben dem Träger erfolgt bei einem Einwirken von Querkräften eine seitliche Abstützung, die den Querkräften entgegengesetzt wirkende Halterungskräfte erzeugen. Hierdurch wird eine Abstützung erreicht, die bei Querkräften in einer Größenordnung, die bei einer üblichen Handhabung gegeben ist, ein Verschieben des Polsterelementes oder ein Trennen oder Abrollen des Polsterelementes vom Träger vermeidet.

Eine besonders wirksame Abstützung kann dadurch erreicht werden, daß der Stützschenkel mindestens bereichsweise am Träger anliegt.

Ein unbeabsichtigtes Abziehen des Polsterelementes vom Träger kann dadurch verhindert werden, daß der Träger einen Hinterschnitt für das Polsterelement ausbildet.

Zur Unterstützung eines geringen Baugewichtes wird vorgeschlagen, daß sich der Hinterschnitt segmentweise erstreckt.

Eine typische Ausführungsform besteht darin, daß das Polsterelement als ein elastomeres Polster ausgebildet ist.

Gemäß einer Ausführungsvariante ist vorgesehen, daß das Polsterelement im Bereich einer Stirnstütze angeordnet ist.

Ebenfalls ist es möglich, daß das Polsterelement im Bereich einer Nasalmaske angeordnet ist.

Ein weiteres Anwendungsgebiet besteht darin, daß das Polsterelement im Bereich einer Vollgesichtsmaske angeordnet ist.

Bei Anwendungen im Bereich von Beatmungsmasken ist insbesondere daran gedacht, daß das Polsterelement mindestens bereichsweise als ein Maskenwulst ausgebildet ist.

Eine besonders hohe Fixiersicherheit kann dadurch erreicht werden, daß der Stützschenkel in eine vom Träger ausgebildete umlaufende Nut eingeführt ist.

Ein Abrollen des Polsterelements vom Träger wird dadurch verhindert, daß das Polsterelement mindestens einen Quersteg aufweist.

Eine Abrollsicherheit bei unterschiedlichen Krafteinwirkungen kann dadurch erreicht werden, daß beidseitig am Stützschenkel Querstege angeordnet sind.

Zur Realisierung des Hinterschnittes zwischen dem Polsterelement und dem Träger ist daran gedacht, daß das Polsterelement die Nut zur Aufnahme des Haltesteges des Trägers aufweist.

Eine nochmals verbesserte Positioniersicherheit wird dadurch erreicht, daß im wesentlichen parallel zum Stützschenkel ein innerer Stützschenkel des Polsterelementes verläuft.

Eine vergleichsweise steife Halterungskonstruktion auch bei Verwendung von elastomeren Werkstoffen kann dadurch erreicht werden, der Stützschenkel und der innere Stützschenkel zur Ausbildung eines U-Profils von einer Schenkelüberleitung miteinander verbunden sind.

In der Zeichnung sind Ausführungsbeispiele der Erfindung schematisch dargestellt. Es zeigen:
- Fig. 1: eine Seitenansicht einer Stirnstütze mit Polsterelement,
- Fig. 2: eine Draufsicht gemäß Blickrichtung II in Fig. 1,
- Fig. 3: einen Querschnitt gemäß Schnittlinie III-III in Fig. 1,
- Fig. 4: eine perspektivische Darstellung der Stirnstütze gemäß Fig. 1 in einem teilweise auseinandergenommenen Zustand,
- Fig. 5: einen Querschnitt gemäß Schnittlinie V-V in Fig. 4 bei ausschließlicher Darstellung des Polsterelementes,
- Fig. 6: den Querschnitt V-V in Fig. 4 bei ausschließlicher Darstellung des Trägers,
- Fig. 7: eine Seitenansicht einer anderen Ausführungsform einer Stirnstütze,
- Fig. 8: eine Draufsicht gemäß Blickrichtung VIII in Fig. 7,
- Fig. 9: einen Querschnitt gemäß Schnittlinie IX-IX in Fig. 7,
- Fig. 10: eine perspektivische Darstellung der Stirnstütze gemäß Fig. 7,
- Fig. 11: eine perspektivische Darstellung einer Nasalmaske,
- Fig. 12: eine weitere perspektivische Darstellung der Nasalmaske gemäß Fig. 11 in einer anderen räumlichen Orientierung,
- Fig. 13: einen Querschnitt gemäß Schnittlinie XIII-XIII in Fig. 12,
- Fig. 14: eine weitere perspektivische Darstellung der Nasalmaske gemäß Fig. 11 in einem teilweise auseinander genommenen Zustand und
- Fig. 15: den Träger und das Polsterelement gemäß Fig. 13 in einem voneinander getrennten Zustand.

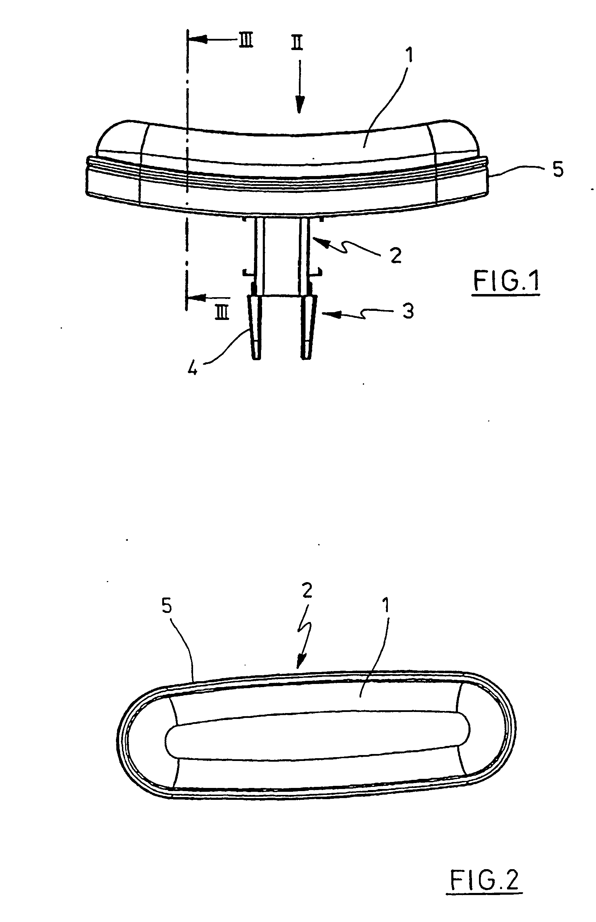

Fig. 1 zeigt eine Ausführungsform, bei der ein Polsterelement (1) im Bereich einer Stirnstütze (2) angeordnet ist, die als Teil einer nicht vollständig dargestellten Beatmungseinrichtung (3) realisiert ist. Die Stirnstütze (2) kann über ein Halterungselement (4) mit einem nicht dargestellten Geräteteil der Beatmungseinrichtung (3) verbunden werden.

Insbesondere ist daran gedacht, das Polsterelement (1) aus einem elastomeren Material zu realisieren. Das Polsterelement (1) wird von einem Träger (5) der Stirnstütze (2) gehaltert, der bei der dargestellten Ausführungsform als ein nutförmiges Bauelement der Stirnstütze (2) realisiert ist.

Aus der Draufsicht gemäß Fig. 2 ist erkennbar, daß der Träger (5) in Form eines langgestreckten Ovales verläuft und ein haubenartiges Polsterelement (1) haltert.

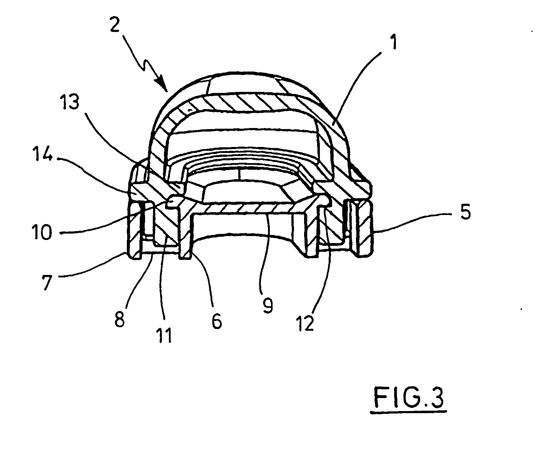

Aus der Querschnittdarstellung in Fig. 3 ist erkennbar, daß der Träger (5) ein im wesentlichen u-förmiges Halterungsprofil ausbildet, das einen Innenschenkel (6) und einen Außenschenkel (7) aufweist. Die Innenschenkel (6) und die Außenschenkel (7) sind von einem Querschenkel (8) miteinander verbunden. Durch die umlaufende Gestaltung des Trägers (5) ist es möglich, im Bereich einer vom Innenschenkel (6) begrenzten Fläche einen Boden (9) anzuordnen. Ausgehend vom Innenschenkel (6) erstreckt sich in Richtung auf den Außenschenkel (7) und etwa auf einem Niveau des Bodens (9) ein quer verlaufender Haltesteg (10).

Das Polsterelement (1) erstreckt sich bei der dargestellten Ausführungsform mit einem Stützschenkel (11) seitlich neben dem Innenschenkel (6) des Trägers (5). Der Haltesteg (10) greift in eine Nut (12) des Polsterelementes (1) ein. Darüber hinaus liegt das Polsterelement (1) mit einem inneren Quersteg (13) auf dem Haltesteg (10) auf. Zu einer weiteren Erhöhung der Positioniersicherheit liegt das Polsterelement (1) mit einem äußeren Quersteg (14) auf dem Außenschenkel (7) des Trägers (5) auf.

Bei der dargestellten Ausführungsform weist der Stützschenkel (11) einen Abstand zum Außenschenkel (7) auf. Es ist aber auch möglich, den Abstand zwischen dem Stützschenkel (11) und dem Außenschenkel (7) kleiner als bei der dargestellten Ausführungsform zu realisieren, darüber hinaus ist es auch möglich, daß der Stützschenkel (11) gegen den Außenschenkel (7) grenzt. Schließlich ist auch daran gedacht, den Stützschenkel (11) entlang des Außenschenkels (7) verlaufen zu lassen und mit einem Abstand zum Innenschenkel (6) anzuordnen.

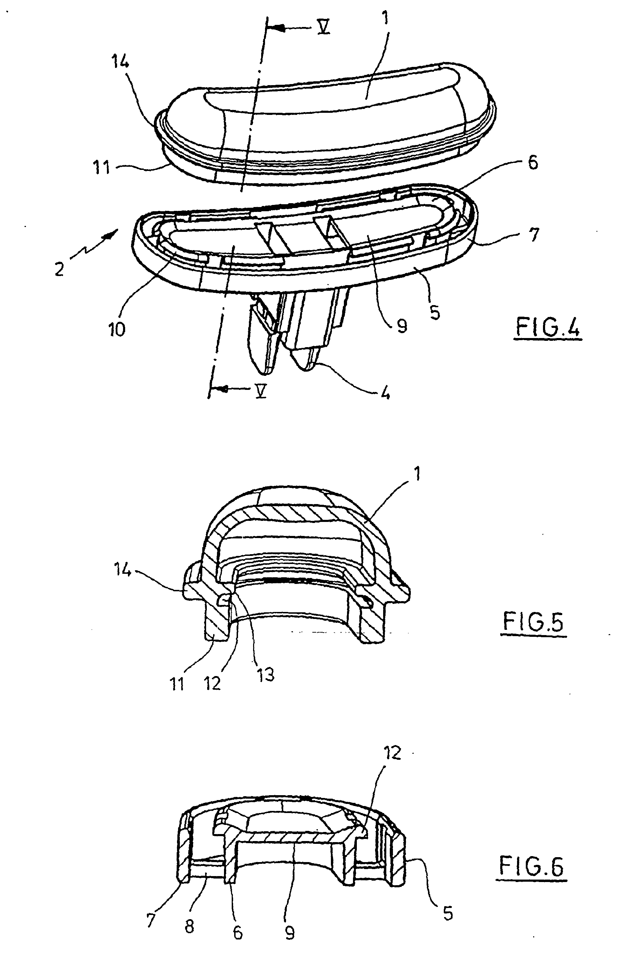

Aus der Darstellung in Fig. 4 ist insbesondere erkennbar, daß der Innenschenkel (6) und der Außenschenkel (7) als umlaufende Wandungen realisiert sind und daß sich der Haltesteg (10) lediglich segmentweise erstreckt. Ebenfalls ist erkennbar, daß bei der dargestellten Ausführungsform der äußere Quersteg (14) des Polsterelementes (1) entlang des äußeren Umfanges des Polsterelementes (1) umläuft.

Fig. 5 veranschaulicht, daß auch der innere Quersteg (13) und die Nut (12) eine umlaufende Gestaltung aufweisen.

Fig. 6 veranschaulicht, daß insbesondere daran gedacht ist, den Haltesteg (10) zur Verbindung des Innenschenkels (6) und des Außenschenkels (7) aus einzelnen Segmenten zu realisieren.

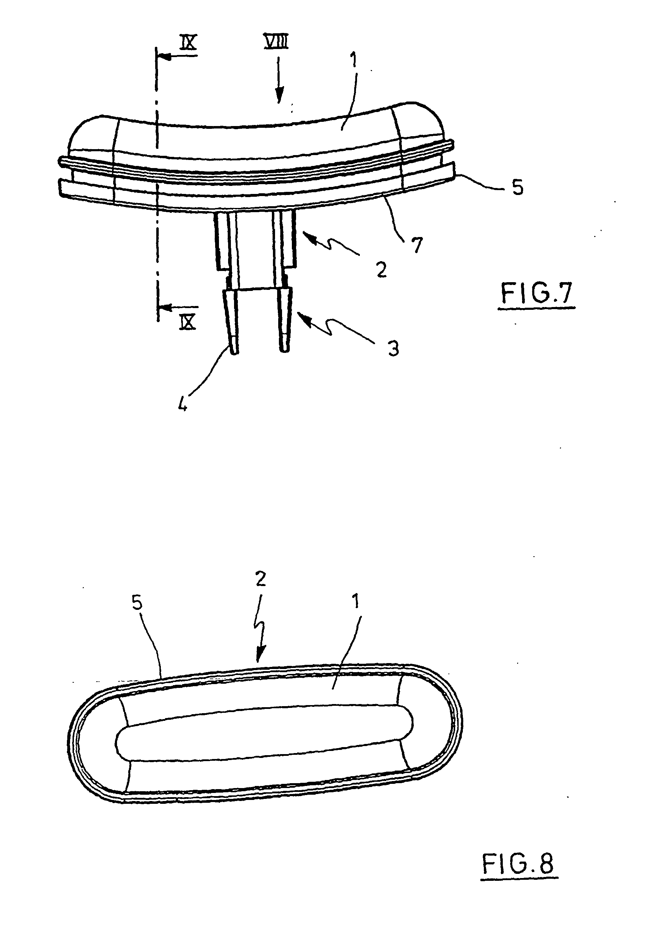

Fig. 7 zeigt eine Ausführungsform der Stirnstütze (2), bei der der Träger (5) mit einem modifizierten Außenschenkel (7) versehen ist.

Die in Fig. 8 dargestellte Draufsicht auf die Stirnstütze (2) gemäß Fig. 7 entspricht im wesentlichen der Draufsicht auf die Stirnstütze (2) gemäß Fig. 1, die in Fig. 2 dargestellt ist.

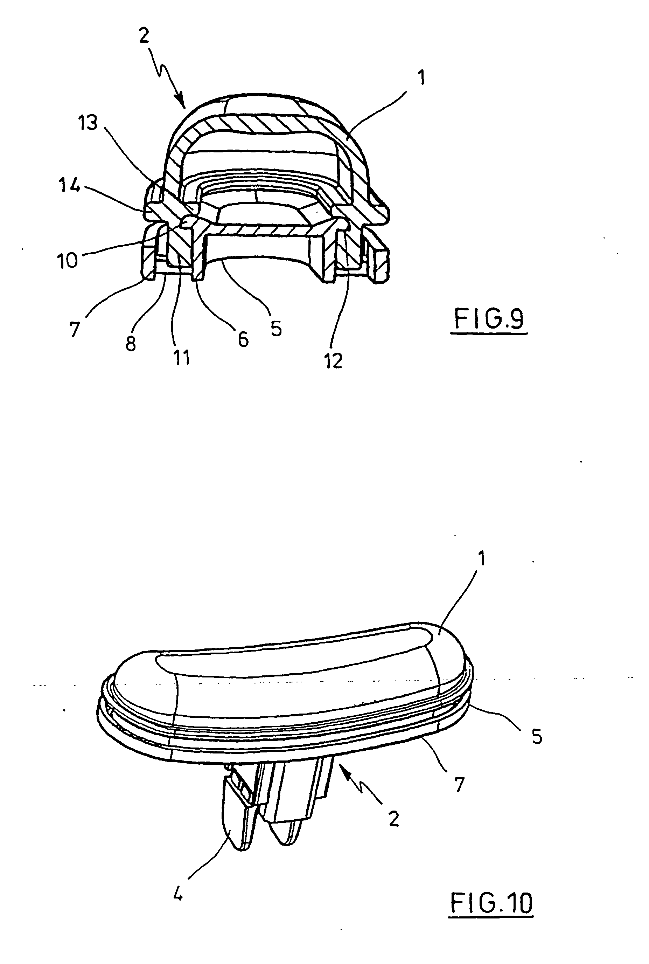

Aus Fig. 9 ist der modifizierte Verlauf des Außenschenkels (7) zu erkennen. Die unterschiedliche Gestaltung fällt insbesondere bei einem Vergleich mit der Darstellung in Fig. 3 auf. Bei der Ausführungsform gemäß Fig. 3 erstreckt sich der Außenschenkel (7) im Bereich seiner dem Querschenkel (8) abgewandten Ausdehnung etwa auf ein Höhenniveau, das dem Höhenniveau des Haltesteges (10) entspricht. Bei der Ausführungsform gemäß Fig. 9 weist der Außenschenkel (7) in dieser Richtung nur eine etwa halb so große Dimensionierung auf. Der äußere Quersteg (14) liegt bei dieser Ausführungsform nicht auf dem Außenschenkel (7) auf.

Fig. 10 veranschaulicht die Stirnstütze gemäß Fig. 8 und Fig. 9 nochmals in einer perspektivischen Darstellung. Auch hier läßt sich die verringerte Dimensionierung des Außenschenkels (7) gut erkennen.

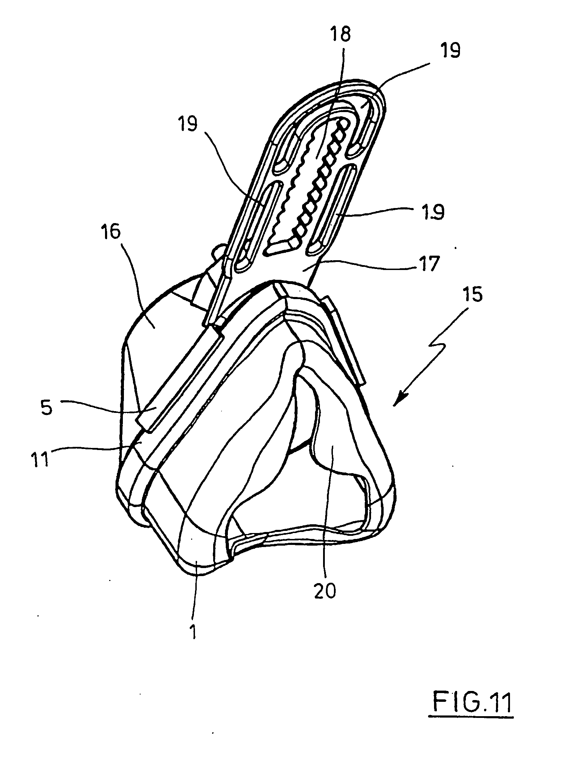

Fig. 11 zeigt eine Ausführungsform, bei der das Polsterelement (1) im Bereich einer Nasalmaske (15) angeordnet ist. Hierdurch soll insbesondere verdeutlicht werden, daß das Halterungsprinzip des Polsterelementes (1) im Bereich des Trägers (5) unter Verwendung eines Stützschenkels (11) bei unterschiedlichen Beatmungseinrichtungen bzw. bei unterschiedlichen Teilen von Beatmungseinrichtungen (3) erfolgen kann. Ein weiteres Anwendungsgebiet besteht beispielsweise bei einem Einsatz des Polsterelementes (1) im Bereich einer Vollgesichtsmaske.

Die Nasalmaske (15) weist einen Grundkörper (16) auf, der im Bereich seiner dem Polsterelement (1) zugewandten Ausdehnung den Träger (5) bereitstellt. Der Grundkörper (16) haltert darüber hinaus eine Befestigungslasche (17) mit Zentralschlitz (18) und Randschlitzen (19).

Zur Erleichterung einer Anpassung an die individuelle Nasenkontur eines Patienten verläuft das Polsterelement (1) im Bereich seiner dem Träger (5) abgewandten Ausdehnung in Form von dünnen Dichtlippen (20).

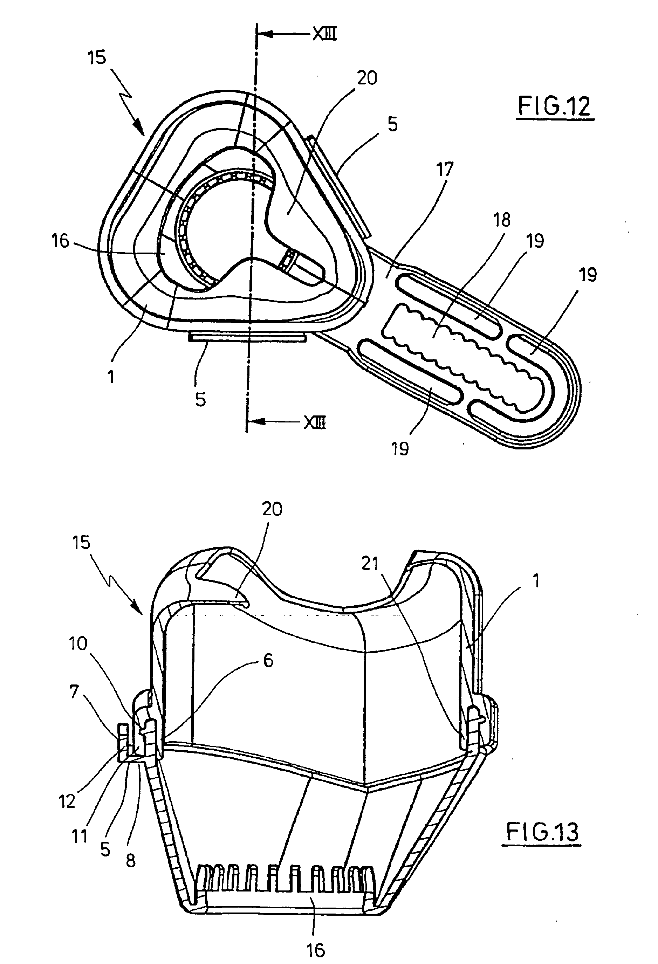

Fig. 12 veranschaulicht, daß der Grundkörper (16) eine gerundet konturierte Grundfläche ähnlich zu einem Dreieck begrenzt. Der Träger (5) ist lediglich segmentweise mit zwei zueinander parallelen Schenkeln realisiert.

Fig. 13 veranschaulicht das Halterungsprinzip des Polsterelementes (1) im Bereich des Trägers (5). Der Innenschenkel (6) des Trägers (5) ist bei dieser Ausführungsform wieder umlaufend realisiert, der Außenschenkel (7) erstreckt sich jedoch nur segmentweise entlang des Umfanges. Ähnlich wie die segmentartige Konstruktion des Verlaufes des Außenschenkels (7) wird auch der Querschenkel (8) zur Verbindung des Außenschenkels (7) mit dem Innenschenkel (6) segmentartig realisiert. Das Polsterelement (1) verläuft mit seinem Stützschenkel (11) am Innenschenkel (6) des Trägers (5) anliegend. Es erfolgt auch bei dieser Ausführungsform ein Eingreifen des quer orientierten Haltesteges (10) in die Nut (12) des Polsterelementes (1). Zusätzlich zum Stützschenkel (11) weist das Polsterelement (1) bei dieser Ausführungsform einen inneren Stützschenkel (21) auf, der sich im wesentlichen parallel zum Stützschenkel (11) erstreckt und an einer Innenfläche des Innenschenkels (6) anliegt. Durch diese Gestaltung bildet das Polsterelement (1) im Bereich des Trägers (5) ein U-Profil aus, in das der Innenschenkel (6) des Trägers (5) eingeführt ist.

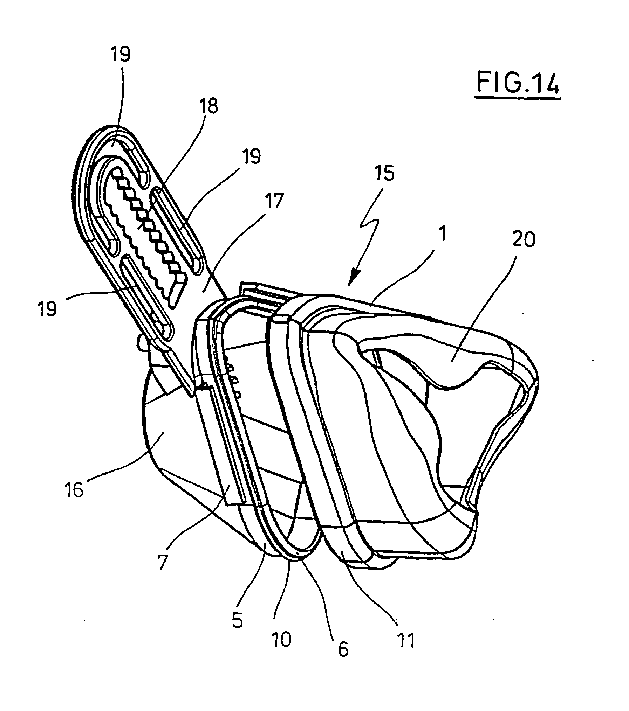

Fig. 14 veranschaulicht in einer anderen perspektivischen Darstellung nochmals die umlaufende Gestaltung des Innenschenkels (6) des Trägers (5) und den segmentweisen Verlauf des Außenschenkels (7). Ebenfalls ist die umlaufende Gestaltung des Stützschenkels (11) des Polsterelementes (1) erkennbar.

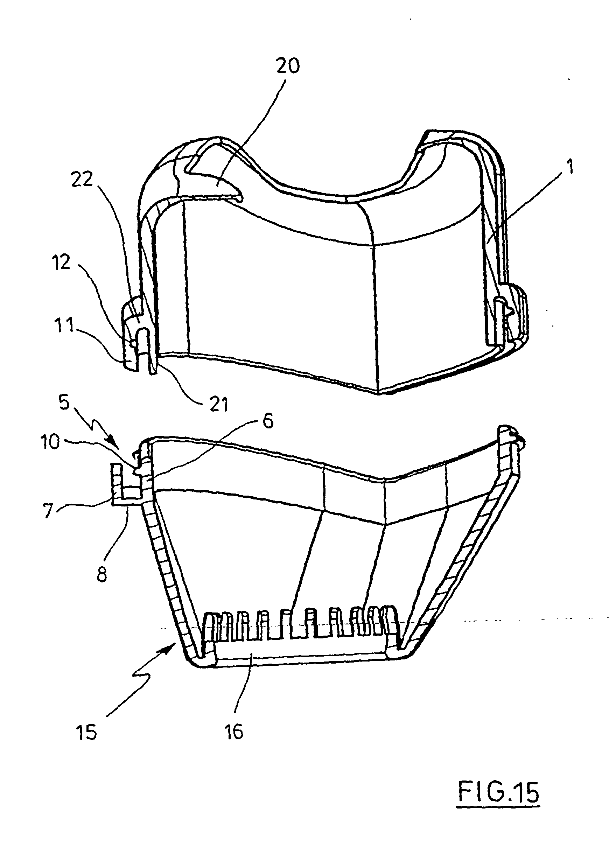

Fig. 15 veranschaulicht die Konstruktion der Nasalmaske (15) in einem Zustand, in dem das Polsterelement (1) vom Grundkörper (16) abgenommen ist. Insbesondere ist hier der Verlauf der beiden für einen gegenseitigen Eingriff vorgesehenen U-Profile erkennbar, wobei das erste U-Profil durch den Innenschenkel (6), den Außenschenkel (7) sowie den Querschenkel (8) des Trägers (5) und das zweite U-Profil durch den Stützschenkel (11), den inneren Stützschenkel (21) sowie eine Schenkelüberleitung (22) des Polsterelementes (1) realisiert ist.

## Patentansprüche

1. Vorrichtung zur Abstützung einer Beatmungseinrichtung relativ zu einem Kopfbereich eines Benutzers, die mindestens ein von einem Träger (5) gehaltertes Polsterelement (1) aufweist sowie bei der sich das Polsterelement (1) mit mindestens einem Stützschenkel (11) mindestens bereichsweise seitlich neben einem Außenschenkel (7) des Trägers (5) erstreckt und mindestens bereichsweise mit einem Abstand zum Außenschenkel (7) ein Innenschenkel (6) des Trägers (5) verläuft, **dadurch gekennzeichnet, daß** sich zwischen dem Stützschenkel (11) und dem Außenschenkel (7) mindestens bereichsweise ein Abstand erstreckt, und daß das Polsterelement (1) eine Nut (12) zur Aufnahme eines Haltesteges (10) des Trägers (5) aufweist, und daß das Polsterelement (1) und der Träger (5) über die Nut (12) und den Haltesteg (10) miteinander verbunden sind.

2. Vorrichtung nach Anspruch 1, **dadurch gekennzeichnet, daß** der Stützschenkel (11) mindestens bereichsweise am Träger (5) anliegt.

3. Vorrichtung nach Anspruch 1 oder 2, **dadurch gekennzeichnet, daß** der Träger (5) einen Hinterschnitt für das Polsterelement (1) ausbildet.

4. Vorrichtung nach Anspruch 3, **dadurch gekennzeichnet, daß** sich der Hinterschnitt segmentweise erstreckt.

5. Vorrichtung nach einem der Ansprüche 1 bis 4, **dadurch gekennzeichnet, daß** das Polsterelement (1) als ein elastomeres Polster ausgebildet ist.

6. Vorrichtung nach einem der Ansprüche 1 bis 5, **dadurch gekennzeichnet, daß** das Polsterelement (1) im Bereich einer Stirnstütze (2) angeordnet ist.

7. Vorrichtung nach einem der Ansprüche 1 bis 5, **dadurch gekennzeichnet, daß** das Polsterelement (1) im Bereich einer Nasalmaske (15) angeordnet ist.

8. Vorrichtung nach einem der Ansprüche 1 bis 5, **dadurch gekennzeichnet, daß** das Polsterelement (1) im Bereich einer vollgesichtsmaske angeordnet ist.

9. Vorrichtung nach Anspruch 7 oder 8, **dadurch gekennzeichnet, daß** das Polsterelement (1) mindestens bereichsweise als ein Maskenwulst ausgebildet ist.

10. Vorrichtung nach einem der Ansprüche 1 bis 9, **dadurch gekennzeichnet, daß** der Stützschenkel (11) in eine vom Träger (5) ausgebildete umlaufende Nut eingeführt ist.

11. Vorrichtung nach einem der Ansprüche 1 bis 10, **dadurch gekennzeichnet, daß** das Polsterelement (1) mindestens einen Quersteg (13) aufweist.

12. Vorrichtung nach einem der Ansprüche 1 bis 11, **dadurch gekennzeichnet, daß** beidseitig am Stützschenkel (11) Querstege (13, 14) angeordnet sind.

13. Vorrichtung nach einem der Ansprüche 1 bis 12, **dadurch gekennzeichnet, daß** im wesentlichen parallel zum Stützschenkel (11) ein innerer Stützschenkel (21) des Polsterelementes (1) verläuft.

14. vorrichtung nach einem der Ansprüche 1 bis 13, **dadurch gekennzeichnet, daß** der Stützschenkel (11) und der innere Stützschenkel (21) zur Ausbildung eines U-Profils von einer Schenkelüberleitung (22) miteinander verbunden sind.

15. Vorrichtung nach einem der Ansprüche 1 bis 14, **dadurch gekennzeichnet, daß** der Stützschenkel (11) mindestens bereichsweise am Außenschenkel (7) anliegt.

16. Vorrichtung nach einem der Ansprüche 1 bis 15, **dadurch gekennzeichnet, daß** der Stützschenkel (11) umlaufend ausgebildet ist.

17. Vorrichtung nach einem der Ansprüche 1 bis 16, **dadurch gekennzeichnet, daß** der Außenschenkel (7) umlaufend ausgebildet ist.

18. Vorrichtung nach einem der Ansprüche 1 bis 17, **dadurch gekennzeichnet, daß** der Quersteg (14) mindestens bereichsweise auf dem Außenschenkel (7) aufliegt.

19. Vorrichtung nach einem der Ansprüche 1 bis 18, **dadurch gekennzeichnet, daß** sich zwischen dem Quersteg (14) und dem Außenschenkel (7) mindestens bereichsweise ein Abstand erstreckt.

## Claims

1. Device for supporting a respirator relative to a user's head region, which device has at least one cushioning element (1) held by a carrier (5) and in which device the cushioning element (1) extends, with at least one supporting sidepiece (11), laterally next to an outer sidepiece (7) on the carrier (5), at least in certain regions, and an inner sidepiece (6) on the carrier (5) extends, at least in certain regions, with a gap in relation to the outer sidepiece (7), **characterised in that** a gap extends, at least in certain regions, between the supporting sidepiece (11) and the outer sidepiece (7), and that the cushioning element (1) has a groove (12) for receiving a retaining rib (10) on the carrier (5), and that the cushioning element (1) and the carrier (5) are connected to one another via the groove (12) and the retaining rib (10).

2. Device according to claim 1, **characterised in that** the supporting sidepiece (11) rests against the carrier (5), at least in certain regions.

3. Device according to claim 1 or 2, **characterised in that** the carrier (5) forms an undercut for the cushioning element (1).

4. Device according to claim 3, **characterised in that** the undercut extends in segments.

5. Device according to one of claims 1 to 4, **characterised in that** the cushioning element (1) is constructed as an elastomer cushion.

6. Device according to one of claims 1 to 5, **characterised in that** the cushioning element (1) is arranged in the region of a forehead support (2).

7. Device according to one of claims 1 to 5, **characterised in that** the cushioning element (1) is arranged in the region of a nasal mask (15).

8. Device according to one of claims 1 to 5, **characterised in that** the cushioning element (1) is arranged in the region of a full-face mask.

9. Device according to claim 7 or 8, **characterised in that** the cushioning element (1) is constructed, at least in certain regions, as a bead on a mask.

10. Device according to one of claims 1 to 9, **characterised in that** the supporting sidepiece (11) is introduced into a circumferential groove formed by the carrier (5).

11. Device according to one of claims 1 to 10, **characterised in that** the cushioning element (1) has at least one transverse rib (13).

12. Device according to one of claims 1 to 11, **characterised in that** transverse ribs (13, 14) are arranged on both sides of the supporting sidepiece (11).

13. Device according to one of claims 1 to 12, **characterised in that** an inner supporting sidepiece (21) on the cushioning element (1) extends substantially parallel to the supporting sidepiece (11).

14. Device according to one of claims 1 to 13, **characterised in that** the supporting sidepiece (11) and the inner supporting sidepiece (21) are connected to one another by a sidepiece transition (22) to form a U-shaped profile.

15. Device according to one of claims 1 to 14, **characterised in that** the supporting sidepiece (11) rests against the outer sidepiece (7), at least in certain regions.

16. Device according to one of claims 1 to 15, **characterised in that** the supporting sidepiece (11) is of circumferential construction.

17. Device according to one of claims 1 to 16, **characterised in that** the outer sidepiece (7) is of circumferential construction.

18. Device according to one of claims 1 to 17, **characterised in that** the transverse rib (14) rests on the outer sidepiece (7), at least in certain regions.

19. Device according to one of claims 1 to 18, **characterised in that** a gap extends between the transverse rib (14) and the outer sidepiece (7), at least in certain regions.

## Revendications

1. Dispositif d'appui d'un appareil respiratoire par rapport à une partie de la tête d'un utilisateur, lequel dispositif présente au moins un élément rembourré (1), maintenu par un support (5), et dans lequel l'élément rembourré (1) s'étend latéralement, par au moins une aile d'appui (11), au moins localement, à côté d'une aile externe (7) du support (5), et une aile interne (6) du support (5) s'étend, au moins localement, à un intervalle de l'aile externe (7), **caractérisé en ce qu'**un intervalle s'étend entre l'aile d'appui (11) et l'aile externe (7), et **en ce que** l'élément rembourré (1) présente, au moins localement, une rainure (12) pour le logement d'une patte de maintien (10) du support (5), et **en ce que** l'élément rembourré (1) et le support (5) sont reliés l'un à l'autre par l'intermédiaire de la rainure (12) et de la patte de maintien (10).

2. Dispositif selon la revendication 1, **caractérisé en ce que** l'aile d'appui (11), appuie, au moins localement, contre le support (5).

3. Dispositif selon revendication 1 ou 2, **caractérisé en ce que** le support (5) présente une contre-dépouille pour l'élément rembourré (1).

4. Dispositif selon la revendication 3, **caractérisé en ce que** la contre-dépouille s'étend par segments.

5. Dispositif selon l'une des revendications 1 à 4, **caractérisé en ce que** l'élément rembourré (1) est réalisé sous la forme d'un rembourrage en élastomère.

6. Dispositif selon l'une des revendications 1 à 5, **caractérisé en ce que** l'élément rembourré (1) est disposé dans la région d'un appui-front (2).

7. Dispositif selon l'une des revendications 1 à 5, **caractérisé en ce que** l'élément rembourré (1) est disposé dans la région d'un masque nasal (15).

8. Dispositif selon l'une des revendications 1 à 5, **caractérisé en ce que** l'élément rembourré (1) est disposé dans la région d'un masque complet.

9. Dispositif selon revendication 7 ou 8, **caractérisé en ce que** l'élément rembourré (1) est configuré, au moins localement, sous la forme d'un bourrelet de masque.

10. Dispositif selon l'une des revendications 1 à 9, **caractérisé en ce que** l'aile d'appui (11) est introduite dans une rainure périphérique, formée par le support (5).

11. Dispositif selon l'une des revendications 1 à 10, **caractérisé en ce que** l'élément rembourré (1) présente au moins une bande transversale (13).

12. Dispositif selon l'une des revendications 1 à 11, **caractérisé en ce qu'**une bande transversale (13, 14) est disposée des deux côtés de l'aile d'appui (11).

13. Dispositif selon l'une des revendications 1 à 12, **caractérisé en ce qu'**une aile d'appui interne (21) de l'élément rembourré (1) s'étend essentiellement parallèlement par rapport à l'aile d'appui (11).

14. Dispositif selon l'une des revendications 1 à 13, **caractérisé en ce que** l'aile d'appui (11) et l'aile d'appui interne (21) sont reliées ensemble par une aile de liaison (22) pour former un profilé en U.

15. Dispositif selon l'une des revendications 1 à 14, **caractérisé en ce que** l'aile d'appui (11) appuie, au moins localement, contre l'aile externe (7).

16. Dispositif selon l'une des revendications 1 à 15, **caractérisé en ce que** l'aile d'appui (11) est formée en périphérie.

17. Dispositif selon l'une des revendications 1 à 16, **caractérisé en ce que** l'aile externe (7) est formée en périphérie.

18. Dispositif selon l'une des revendications 1 à 17, **caractérisé en ce que** la bande transversale (14) appuie, au moins localement, contre l'aile externe (7)

19. Dispositif selon l'une des revendications 1 à 18, **caractérisé en ce qu'**un intervalle s'étend, au moins localement, entre la bande transversale (14) et l'aile externe (7).
